(19) Europäisches Patentamt
European Patent Office
Office européen des brevets

(11) Numéro de publication: **0 228 978 B1**

# (12) FASCICULE DE BREVET EUROPEEN

(45) Date de publication de fascicule du brevet: **17.04.91**

(51) Int. Cl.$^5$: **A61K 31/695**, A61K 33/04, //(A61K33/04,31:695)

(21) Numéro de dépôt: **86450026.9**

(22) Date de dépôt: **22.12.86**

## (54) Compositions à usage thérapeutique comprenant des composés organo-siliciés.

(30) Priorité: **23.12.85 FR 8519204**

(43) Date de publication de la demande: **15.07.87 Bulletin 87/29**

(45) Mention de la délivrance du brevet: **17.04.91 Bulletin 91/16**

(84) Etats contractants désignés: **AT BE CH DE ES GB GR IT LI LU NL SE**

(56) Documents cités:
**FR-A- 2 158 068**
**FR-A- 2 160 293**
**FR-A- 2 230 376**

**UNLISTED DRUGS, vol. 31, no. 4, avril 1979, Chatham, New Jersey, US**

(73) Titulaire: **Le Ribault, Loic**
**23, Chemin des Dames**
**F-33260 La-Teste-Du-Buch(FR)**

Titulaire: **Bonnaval-Lamothe, Michel**
**Côte-Belle**
**F-33410 Cadillac sur Garonne(FR)**

Titulaire: **Nardou, René**
**32, Allée Emile Pereire**
**F-33120 Arcachon(FR)**

(72) Inventeur: **Le Ribault, Loic**
**23 Chemin des Dames**
**F-33260 La Teste de Buch(FR)**

(74) Mandataire: **Thébault, Jean-Louis**
**Cabinet Thébault S.A. 50 Cours de Verdun**
**F-33000 Bordeaux(FR)**

**EP 0 228 978 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

**Description**

La présente invention concerne de nouvelles compositions è usage thérapeutique, notamment utiles pour le traitement de l'asthme et des phénomènes allergiques en général, ainsi que de la sinusite, de l'herpès et de l'hépatite virale, du zona et des maladies virales en général, chez l'homme.

Les composés organo-siliciés sont déjà utilisés en thérapeutique humaine depuis de nombreuses années.

Ces composés hydrosolubles et atoxiques traversent aisèment l'épiderme et le derme par application locale (GUEYNE, DUFFAUT et QUILICHINI, Thérapie 1962, 17 , 417).

Les nombreuses propriétés thérapeutiques de ces composés organo-siliciés, utilisés seuls, ont été exposées dans plusieurs brevets (en particulier les brevets français publiés sous les N° 2.158.068, 2.160.293 et 2.230.376).

L'administration de ces composés organo-siliciés se fait, selon ces documents, par voie intramusculaire ou intraveineuse, ou encore par électrophorèse, le principe actif étant alors en solution dans l'eau (solution isotonique), éventuellement additionnée d'un alcool et d'un polyalcool, tel que le glycérol, et/ou d'un sel de sodium d'un acide organique pharmaceutiquement acceptable.

Il avait été déjà démontré, en particulier dans les brevets mentionnés ci-dessus, que certaines substances de natures diverses pouvaient potentialiser et élargir le spectre d'action de ces composés organo-siliciés.

On a maintenant trouvé de façon inattendue qu'on peut réaliser une nouvelle composition à usage thérapeutique en combinant, dans des proportions appropriées, un ou plusieurs composés organo-siliciés avec de l'hyposulfite (ou thiosulfate) de sodium ou de magnésium.

On a également trouvé que les compositions de ce type sont tout particulièrement utiles contre l'asthme et les phénomènes allergiques en général, la sinusite, l'herpès et l'hépatite virale, le zona et les maladies virales en général, contre lesquels elles exercent une action spécifique, et que les compositions ainsi réalisées peuvent être administrées simplement par voie transcutanée, chez l'homme ou l'animal.

L'invention a donc pour premier objet une composition à usage thérapeutique, notamment utile pour le traitement de l'asthme, de la sinusite, de l'herpès et de l'hépatite virale entre autres, caractérisée en ce qu'elle comprend, en solution aqueuse :

(1) au moins un composé organo-silicié répondant à l'une quelconque des deux formules

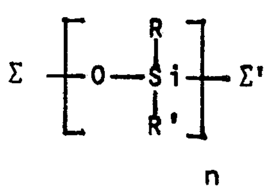

ou

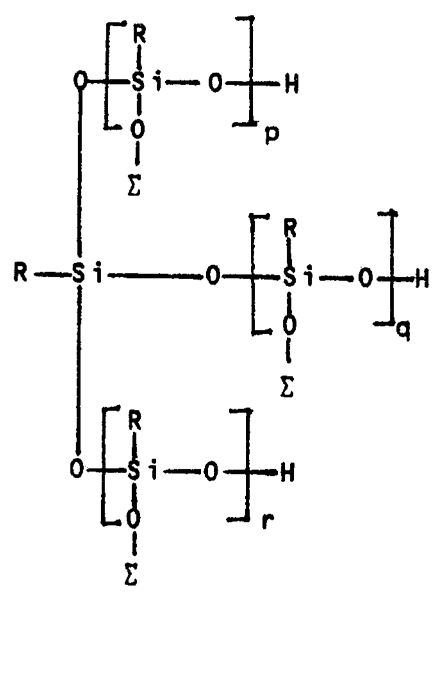

(A) (B)

: dans lesquelles :

- n est un nombre entier compris entre 1 et 1000 et, de préférence, entre 1 et 50 ;

- p, q, r sont des nombres entiers compris entre 0 et 1000 et, de préférence, entre 0 et 60 ;
- R et R' représentant, indépendamment l'un de l'autre, un groupement aliphatique linéaire, ramifié ou cyclique, saturé ou non, et comportant éventuellement des hétéroatomes, ou aromatique, hétéroaromatique, arylaliphatique,ou hétéroarylaliphatique, éventuellement substitués par des groupes fonctionnels ; R pouvant aussi être OH, OR ou $OSiR_3$.
- Σ étant R ou H ou $SiR_3$ ;
- Σ' étant choisi parmi R et plus particulièrement $CH_3$ ou OR, ou OH ou $OSiR_3$ (où R est tel que défini ci-dessus), ou les sels de métal alcalin ou d'addition avec un acide pharmaceutiquement acceptable, dudit composé, le(s) composé(s) organo-silicié(s) étant utilisé(s) en concentration dans de l'eau variant de $10^{-5}$ à $10^{-1}$ mol/l, et plus particulièrement de $10^{-4}$ à $10^{-2}$ mol/l, et

(2) de l'hyposulfite de sodium ($Na_2S_2O_3.5H_2O$) ou de magnésium, le rapport de (1) à (2) étant de 5 à 50g d'hyposulfite pour 1 litre de solution organo-siliciée, et de préférence d'environ 20 g/litre de solution organo-siliciée.

En variante, la composition selon l' invention peut également comprendre, en une proportion appropriée pour neuttreliser et éventuellement stabiliser ces composés, un ou plusieurs acides des orgoniques (ou leurs sels, en particulier leurs sels alcalins), tels que par exemple l'acide salicylique, l'acide citrique, l' acide propionique, l'acide aspartique, l'acide glutarique et/ou l'acide glutamique. Il y a toutefois lieu de noter que l'acide salicylique doit être proscrit pour le traitement de patients allergiques aux phénols et à l'acide salicylique en particulier et que, dans la mesure où ce cas peut être rencontré, il est alors tout à fait possible de remplacer cet acide par de l'acide citrique ou ses sels, comme il est montré plus loin dans les exemples.

L 'acide organique ou son sel est utilisé en concentrations variant de $10^{-5}$ à $10^{-1}$ mol/l particulièrement de $10^{-2}$ à $10^{-4}$ mol/l.

Dans tous les cas, il est apparu préférable que la solution ait un pH compris entre 3 et 7, ou mieux entre 3 et

Les composés organo-siliciés correspondant à formule susdite sont des produits connus en soi.

On peut utiliser pour la réalisation des compositions selon l'invention, soit ceux qui sont disponibles dans le commerce, soit leurs précurseurs connus, qui les engendrent par hydrolyse, tels que par exemple des silazanes ou alcoxysilanes correspondants (voir en particulier le document FR-A-2.230.376 à ce sujet).

L'hyposulfite de sodium ou de magnésium peut être combiné avec les composés organo-siliciés par addition à la solution de ceux-ci, soit à l'état solidê, soit sous forme d'uns solution aqueuse convenablement dosée, et cela, soit dès l'origine, soit en cours d'application et en particulier lors de l'utilisation à des fins thérapeutiques de la composition selon l'invention.

La composition selon l'invention peut donc consister soit en un produit unique contenant le composé organo-silicié et l'hyposulfite, soit en un produit bi-composants contenant séparément le composé organo-silicié et l'hyposulfite, dont le mélange peut alors être fait extemporanément.

La composition peut être mise à la disposition des utilisateurs dans des flacons, ou dans des ampoules qui constituent des doses unitaires contenant une quantité de produit dosée pour chaque administration.

Les doses unitaires de compositions totale préconisées pour chaque administration transcutanée sont avantageusement d'environ 10 $cm^3$.

Les compositions peuvent être complétées par des agents de neutralisation et/ou de conservation compatibles avec les principes actifs, tels que ceux indiqués plus haut, ainsi que par d'autres constituants tels que des colorants, des parfums ou d'autres excipients, que l'homme du métier est apte à choisir et à incorporer selon ses connaissances en la matière, en fonction des besoins.

A partir de tests effectués sur l'homme, on a pu établir que l'apport de composés organo-siliciés associé à de l'hyposulfite de sodium ou de magnésium sous la forme d'applications sur la peau, par exemple par tamponnage, de compositions conformes à l'invention, a une action transcutanée et exerce une action pharmacologiquement efficace contre les crises d'asthme et les phénomènes allergiques en général, la sinusite, l'herpès, l'hépatite virale, le zona et les maladies virales en général.

Les résultats des essais montrent que les compositions conformes à l'invention sont particulièrement indiquées et exceptionnellement efficaces sans présenter de toxicité notable (comme on le montrera plus loin), pour le traitement et/ou la prévention de l'asthme, des céphalées et des phénomènes allergiques en générale, de la sinusite, de l'herpès, de l'hépatite virale, du zona et des maladies virales en général.

A titre indicatif, on peut recommander comme posologie:

- L'application d'une compresse de coton (environ 5 cm sur 7 cm) imbibée de la composition selon l'invention au niveau de la zone douloureuse ou déficiente ou de toute autre partie du corps.
  La compresse est recouverte d'une feuille de matière plastique, ou de tout autre matière susceptible de maintenir l'humidité en empêchant l'évaporation. La compresse est appliquée pendant une durée

comprise entre 8 et 12 heures par jour (au cours de la nuit par exemple).

- Le massage avec une composition selon l'invention, mise sous forme de crème selon une technique connue.
- Un tamponnage de zone cutanée avec un tampon imprégné de la composition selon l'invention. Ce tamponnage est effectué 2 à 4 fois par jour, par exemple sur les avant-bras, à l'endroit où les vaisseaux sont les plus apparents. Il a en effet été démontré que la composition selon l'invention traversait facilement l'épiderme.
- On peut aussi appliquer simultanément la composition selon l'invention par ionocinèse pratiquée localement au niveau (ou non) de la zone déficiente ou douloureuse. L'appareil pour ionocinèse est constitué d'un générateur de courant continu (intensité de l'ordre de 10-15 milliampères), muni d'un dispositif permettant d'établir ou d'interrompre le courant d'une manière progressive et capable de maintenir une intensité constante pendant la durée de la séance.
  L'intensité du courant est progressivement amenée à 10mA et maintenue durant 20 minutes environ avant d'être abaissée progressivement, puis annulée.
  Dans certains cas, on peut amener l'intensité du courant jusqu'à 25mA, la durée de la séance pouvant aller jusqu'à 30 minutes.
  Le générateur est relié à deux électrodes de carbone ou d'autres matériaux couramment utilisés lors des traitements par ionocinèse. La surface de l'électrode, bien que pouvant être très variable, est couramment de l'ordre de 200 $cm^2$.
  Chacune des deux électrodes est entourée de coton hydrophile que l'on imprègne de la composition selon l'invention.
  L'électrode reliée au pôle négatif du générateur est appliquée sur une partie quelconque du corps, mais de préférence au niveau de la zone déficiente ou douloureuse : elle est maintenue au contact de la peau par un bandage. L'autre électrode, reliée au pôle positif du générateur, peut être appliquée à un endroit quelconque de la peau ou simplement tenue à la main, la surface de contact étant la plus élevée possible. Dans certains cas particuliers, on inverse la polarité des électrodes en cours de séance.
  Il convient de signaler qu'un traitement nécessite 10 à 30 séances espacées de 24 à 72 heures, le rythme le plus couramment adopté étant 2 à 3 séances par semaine.
  Le traitement est totalement indolore. Toutefois, certains sujets sensibles ont une sensation de picotement au niveau des électrodes au cours des séances. Il n'a été observé aucune réaction locale ou générale de l'organisme, si ce n'est un léger érythème au niveau de la zone d'application des électrodes.
  Cependant, lorsque le sujet présente une allergie aux dérivés phénoliques, l'on doit choisir une solution ne contenant pas ce type de dérivés.
- Des injections intramusculaires peuvent également être pratiquées.
  Pour apprécier la toxicité aiguë d'une composition de méthylsiliconate de potassium + hyposulfite de sodium selon l'invention, on a procédé à l'expérimentation relatée ci-après: La composition administrée se présentait sous la forme d'une solution aqueuse à 1,5% (en poids/volume). L'expérimentation a consisté à déterminer la $DL_{50}$ de la composition sur des souris femelles, par administration sous-cutanée. Elle s'est déroulée dans les conditions exposées ci-dessous :

**I - Animaux**

150 souris femelles de race swiss d'un poids moyen de 30g ont été prélevées sur un lot d'animaux reçu depuis plus de quinze jours, donc bien adaptées à leur nouvelle animalerie.
On les a réparties en 6 lots de 25 animaux et numérotées de 1 à 25 dans chaque lot, les lots étant eux-mêmes indexés par les lettres A à F.
Les conditions d'animalerie étaient les suivantes :

. nourriture et boisson: ad libitum
. type d'alimentation: aliment complet du commerce (Extra Labo)
. température moyenne: 21 °C
. hygrométrie: 73%
. heure de début d'expérience: 9 heures

**II - Doses administrées**

A partir d'une expérience préliminaire effectuée sur quelques animaux, on a pu établir que des doses comprises entre 30 et 40 ml/kg présentaient une létalité plus ou moins importante et il en est résulté que les doses choisies ont été les suivantes:

. 25 ml/kg, soit 375 mg/kg de méthylsiliconate de potassium + hyposulfite de sodium pour le groupe A ;
. 30 ml/kg, soit 450 mg/kg de méthylsiliconate de potassium + hyposulfite de sodium pour le groupe B ;
. 35 ml/kg, soit 525 mg/kg de méthylsiliconate de potassium + hyposulfite de sodium pour le groupe C ;
. 40 ml/kg, soit 600 mg/kg de méthylsiliconate de potassium + hyposulfite de sodium pour le groupe D ;
. 45 ml/kg, soit 675 mg/kg de méthylsiliconate de potassium + hyposulfite de sodium pour le groupe E ;
. 50 ml/kg, soit 750 mg/kg de méthylsiliconate de potassium + hyposulfite de sodium pour le groupe F.

La voie d'administration choisie était la voie sous-cutanée.

**III - Résultats**

. LOT A : Aucune mortalité, ni symptôme extérieur de toxicité ne sont apparus, ni immédiatement après l'administration, ni par la suite, c'est-à-dire pendant les quinze jours de surveillance auxquels ont été soumis les animaux. Le comportement et l'état général de ceux-ci étaient tout à fait normaux.

. LOT B : Tout comme pour le lot A, on n'a observé ni mortalité, ni troubles du comportement. L'état général était excellent pendant la période consécutive à l'administration de la composition de méthylsiliconate de potassium + hyposulfite de sodium.

. LOT C : 6 animaux de ce groupe sont morts dans des circonstances comparables et sur une période de temps allant de 45 minutes à 90 minutes après l'administration. Les signes cliniques ante-mortem étaient une prostration, un besoin d'isolement, un ternissement du poil et des crises de tremblement (plus ou moins violentes, mais sans convulsion) débutant 15 à 20 minutes avant la mort, qui a été précédée de quelques soubresauts plus accentués.
L'examen nécroptique a montré qu l'ensemble des organes internes présentait un aspect normal ; en particulier le foie, la rate, le pancréas, les reins et les capsules surrélnales ne révélaient pas d'anomalies visibles. On a pu noter cependant que la vessie était régulièrement vide et que le coeur était arrêté en systole. Les poumons étaient légèrement rosés. Aucun signe particulier n'est apparu ou point d'injection.
Les animaux survivants, dans leur majorité, n'ont pas paru affectés ppar l'administration du produit. Trois ont manifesté de légers signes d'indisposition qui se sont rapidement estompés. Toutefois, tous les animaaux de ce groupe ont présenté une fourrure terne pendant 24 heures. Ensuite, le comportement et l'état général des animaux survivants sont redevenus complètement normaux pendant les quinze jours suivant l'administration.

. LOT D : 11 animaux de ce gupe sont morts dans des conditions et des délais identiques à ceux du groupe C. Ils présentaient des signes cliniques ante-mortem et des examens anatomiques post-mortem tout à fait comparables à ceux-ci. Quatre animaux ont présenté des signes transitoires d'indisposition. Tous les animaux survivants ont présenté une robe terne pendant 24 heures ; ensuite, leur comportement et leur état général ont été parfaitement normaux pendant la période d'observation.

. LOT E : 14 animaux de ce groupe sont morts dans des conditions et des délais identiques à ceux des groupes précédents, avec des tableaux cliniques et anatomiques tout à fait comparables.
Deux animaux ont manifesté une gêne passagère. Tous les survivants ont présenté un poil terne pendant 24 heures. Ensuite, comme pour les survivants des lots C et D, on a pu observer une normalisation complète du comportement et de l'état général pendant toute la période d'observation.

. LOT F : Dans ce groupe 17 animaux sont morts dans des circonstances et des temps comparables à ceux des groupes C,D et E.
Les comportements ante-mortem observés et les examens nécroptiques pratiqués ont permis des observations exactement calquées sur celles des groupes précédents. Quatre animaux ont présenté des signes de gêne passagère. Tous les survivants ont eu une fourrure terne pendant 24 heures. Ils ont ensuite récupéré un comportement et un état général tout à fait normaux pendant la période

d'observation.

A la suite de la période d'observation de quinze jours, tous les animaux survivants de cette expérimentation étaient en vie et en bonne santé après une période d'un mois.

## IV - Discussion et interprétation des résultats

A partir des résulats de cette expérimentation, on a pu calculer le pourcentage de mortalité en fonction de la dose administrée, soit :

lot A létalité : 0%
lot B létalité : 0%
lot C létalité : 24%
lot D létalité : 42%
lot E létalité : 56%
lot F letalité : 67%

La $DL_{50}$ sous-cutanée souris se trouvait donc bien encadrée par deux points en dessous et deux points au-dessus de sa valeur. Le diagramme de probit/log (pourcentage de létalité en fonction du logarithme de la dose en mg/kg) tracé à partir de ces résultats a permis de situer la valeur de cette $DL_{50}$ à 645 mg/kg pour la composition selon l'invention testée.

D'autre part, il a été possible de faire ressortir une limite de toxicité pour l'apparition des premières manifestations toxiques réversibles, telles que la somnolence, le besoin d'isolement. Cette limite a pu être évaluée à 380 mg/kg.

## V - Conclusion

Les compositions selon l'invention apparaissent bien comme peu toxiques, puisque les doses qu'il est préconisé d'utiliser à des fins thérapeutiques sont cent fois moins élevées que la dose provoquant les premières manifestations toxiques.

L'invention est décrite et illustrée plus en détail dans les exemples ci-après, qui ne la limitent aucunement et qui regroupent les indications concernant les compositions et leur préparation et les propriétés révélées par des essais pharmacologiques se rapportant au traitement de diverses maladies chez l'homme.

## EXEMPLE 1

J. R... présentait une kératite herpétique avec ulcération de la cornée, larmoiement intense et forte vasodilatation de la conjonctive, traitée sans résultat par des collyres cortisoniques.

Pendant trois jours, deux bains d'yeux quotidiens lui ont été appliqués avec une solution selon l'invention composée de la façon suivante :

Dans un bécher de 1 litre, on a introduit 1 g d'acide salicylique et on y a ajouté 700 ml d'eau distillée.

On a versé lentement sur cette solution, en agitant énergiquement, 1,2 ml d'une solution de méthylsiliconate de potassium à 45% d'extrait sec.

On a amené le pH de la solution à 4,7 et on a complété à 1 litre avec de l'eau distillée. 20 g d'hyposulfite de sodium ont été ajoutés ensuite.

Suite à l'application de ce traitement, avec une solution rendue isotonique et tamponnée, l'inflammation disparaissait en 24 heures et la cicatrisation s'est avérée être complète au bout de 7 jours.

Revu trois mois après, le malade ne présentait aucune séquelle.

## EXEMPLE 2

Madame L. Z... présentait d'une manière très fréquente (environ deux fois par mois) une éruption herpétique de la lèvre supérieure.

Une série d'applications locales au niveau de la lésion a été réalisée à l'aide d'un coton imbibé d'une solution de la composition selon l'invention, fabriquée de la façon suivante :

Dans un bécher de 1 litre, on a introduit 1 g d'acide citrique et on y a ajouté 700 ml d'eau distillée. On

a versé lentement sur cette solution, en agitant énergiquement, 1,7 ml d'une solution de méthylsiliconate de potassium à 45% d'extrait sec. On a amené le pH de la solution à 4,7 et on a complété à 1 litre avec de l'eau distillée. 20 g d'hyposulfite de sodium ont été finalement ajoutés à l'ensemble.

A raison de trois applications locales de la composition susdite l'aide d'un coton imbibé de cette dernière, une cicatrisation sans trace apparente a pu être constatée au bout de 7 jours, et on a pu établir l'absence de toute récidive avec un recul de trois mois.

**EXEMPLE 3**

Monsieur M. L... était atteint d'herpès génital récidivant qui intéressait le gland et le prépuce.

Une série d'applications locales au niveau de la lésion a été réalisée en utilisant une solution de la composition selon l'invention, fabriquée de la façon suivante: Dans un bécher de 1 litre, on a introduit 1 g d'acide citrique et on a ajouté 800 ml d'eau distillée. On a versé lentement sur cette solution, en agitant énergiquement, 2,5 ml de méthylsiliconate de sodium à 30% d'extrait sec. On a amené le pH de la solution à 4,7, complété à 1 litre avec de l'eau distillée et ajouté 20 g d'hyposulfite de sodium.

Trois applications locales quotidiennes ont été réalisées par tamponnement à l'aide d'un coton imbibé de la solution décrite ci-dessus.

Après une semaine de ce traitement, on constatait la disparation de l'éruption, sans trace apparente. Au bout de trois mois, le malade ne présentait aucune récidive.

**EXEMPLE 4**

Mademoiselle L. B..., atteinte d'herpés récidivant de la vulve, présentait chaque mois pendant la semaine précédant ses règles une éruption douloureuse avec un important oedème intéressant les petites et les grandes lèvres.

Une série d'applications locales au niveau de la lésion a été réalisée en utilisant de la composition selon l'invention, fabriquée de la façon suivante :

Dans un bécher de 1 litre contenant 500 ml d'eau distillée, préalablement refroidie par addition de glace pilée, on a introduit lentement, sous forte agitation, 0,65 g de diméthylchlorosilane et 0,85 g de bicarbonate de sodium. On a vérifié que le pH était voisin de 7. La solution ainsi obtenue était homogène.

On a ajouté lentement, sous forte agitation, 1,1 g de salicylate de sodium. On a amené le pH de la solution à 4,7, puis complété à 1 litre avec de l'eau distillée et finalement ajouté 20 g d'hyposulfite de sodium.

Deux applications locales quotidinennes ont été réalisées pendant la phase éruptive, par tamponnement à l'aide d'un coton imbibé de la solution décrite ci-dessus. La malade a épouvé un soulagement très rapide, et l'oedème a disparu en quelques jours.

Les trois mois suivants, aucune éruption ne s'est produite au moment de la période pré-menstruelle.

**EXEMPLE 5**

Monsieur R. B...présentait un ictère d'apparition brutale. Les examens de laboratoire ont confirmé le diagnostic d'hépatite virale. Les transaminases étaient élevées : 1.100.

On a appliqué au niveau du foie une compresse de coton recouverte de plastique et imbibée de la composition selon l'invention, décrite ci-dessus dans l'exemple 3. Cette compresse a été maintenue dix heures par jour.

Au bout de six jours, l'ictère avait nettement régressé, et les transaminases avaient chuté à la valeur 50.

Après deux mois, le malade ne présentait plus aucun signe clinique ni biologique, d'hépatite.

**EXEMPLE 6**

Monsieur E. F... présentait des crises de dyspnées nocturnes avec suffocations.

A l'aide d'un coton imbibé de la composition selon l'invention, décrite ci-dessus dans l'exemple 1, sept applications par tamponnement lui ont été faites au niveau des avant-bras, le soir au coucher. Ces applications étaient associées à des instillations nasales de la même solution.

On a pu constater que les crises diminuaient d'intensité au bout de 4 jours et disparaissaient progressivement.

Un traitement d'entretien d'une application par semaine a été poursuivi pendant deux mois. Il a été en outre conseillé au malade, pendant cette période, de procéder au tamponnement (associé à l'instillation nasale) à la moindre gêne respiratoire, et, ce, à titre préventif.

Au bout de trois mois, le malade ne présentait plus de crises et n'a pas présenté la moindre récidive depuis huit mois.

**EXEMPLE 7**

Madame B. P... avait passé une série d'examens ayant révélé une hépatite virale. Parmi ceux-ci, les examens chimiques du sang donnaient notamment les résultats suivants :
Transaminases : S.G.0.T. : 1290 UI/l
S.G.P.T. : 2170 UI/l

La malade a alors subi un traitement au moyen de la composition selon l'invention telle que décrite ci-dessus dans l'exemple 1, sous la forme de massages, 4 fois par jour, au niveau du foie, à l'aide d'une crême élaborée avec cette composition.

Cinq jours plus tard les résultats de l'analyse de sang étaient les suivants :
Transaminases : S.G.0.T. : 50 UI/l
S.G.P.T. : 210 UI/L

**EXEMPLE 8**

Madame Y. L... souffrait de sinusite depuis plusieurs dizaines d'années. Durant cette période elle avait pourtant subi plusieurs opérations (extraction des polypes, ablation des amygdales) et divers traitements et examens, sans résultat durable.

Après une nouvelle crise importante, sur laquelle les traitements classiques n'ont donné aucun résultat notable, la malade a reçu une première application de composition selon l'invention telle que décrite dans l'exemple 2 ci-dessus, par frottement au niveau de la racine du nez à l'aide d'une crème élaborée à partir de cette composition. Cette application a été répétée 4 fois par jour. Une heure après la première application ont débuté des écoulements nasaux qui ont obligé la patiente à se moucher presque sans interruption jusqu'au soir. Dès le lendemain, la patiente se sentait "dégagée" et respirait normalement. Aucune nouvelle crise de sinusite n'a été enregistrée depuis lors.

**Revendications**

1. Composition à usage thérapeutique, notamment utile pour le traitement de l'asthme et des phénomènes allergiques en général, de la sinusite, de l'herpès et de l'hépatite virale, du zona et des maladies virales en général, caractérisée en ce qu'elle comprend, en solution aqueuse :

   (1) au moins un composé organo-silicié répondant à l'une quelconque des deux formules :

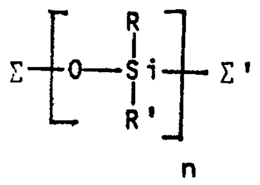

ou

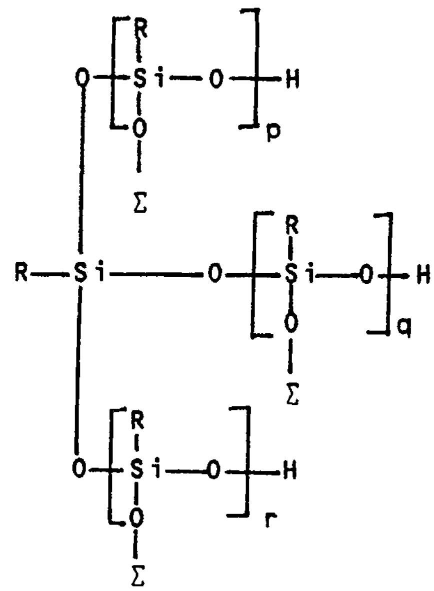

(A) (B)

dans lesquelles :

- n est un nombre entier compris entre 1 et 1000 et, de préfèrence, entre 1 et 50 ;
- p, q, r sont des nombres entiers compris entre 0 et 1000 et, de préférence, entre 0 et 60 ;
- R et R′ représentant, indépendamment l'un de l'autre, un groupement aliphatique linéaire, ramifié ou cyclique, saturé ou non, et comportant éventuellement des hétéroatomes, ou aromatisée, hétéroaromatique, arylaliphatique,ou hétéroarylaliphatique, éventuellement substitués par des groupes fonctionnels ; R pouvant aussi être OH, OR ou $OSiR_3$.
- Σ étant R ou H ou $SiR_3$ ;
- Σ′ étant choisi parmi R et plus particulièrement $CH_3$ ou OR, ou OH ou $OSiR_3$ (où R est tel que défini ci-dessus), ou les sels de métal alcalin ou d'addition avec un acide pharmaceutiquement acceptable, dudit composé, le(s) composé(s) organo-silicié(s) étant utilisé(s) en concentration dans de l'eau variant de $10^{-5}$ à $10^{-1}$ mol/l et plus particulièrement de $10^{-4}$ à $10^{-2}$ mol/l, et

(2) de l'hyposulfite de sodium ($Na_2S_2O_3.5H_2O$) ou de magnésium, le rapport de (1) à (2) étant de 5 à 50g d'hyposulfite pour 1 litre de solution organo-siliciée, et de préférence d'environ 20 g/litre de solution organo-siliciée.

2. Composition selon la revendication 1, caractérisée en ce qu'elle comprend, en outre, un ou plusieurs acides organiques ou leurs sels.

3. Composition selon l'une des revendications 1 ou 2, caractérisée en ce qu'elle consiste en un produit unique contenant le composé organo-silicié et l'hyposulfite dans une seule solution.

4. Composition selon l'une des revendications 1 ou 2, caractérisée en ce qu'elle consiste en un produit bi-composants contenant séparément le composé organo-silicié et l'hyposulfite qui sont destinés à être administrés en même temps.

5. Composition selon l'une quelconque des revendications 1 à 4, caractérisée en ce qu'elle est sous forme de doses unitaires, en flacons ou en ampoules, contenant environ 10 $cm^3$ de ladite composition.

6. Composition selon l'une quelconque des revendications 1 à 5, caractérisée en ce qu'elle présente un pH ajusté entre 3 et 7, de préférence entre 3 et 5.

## Claims

1. Composition for therapeutic use, particularly useful for the treatment of asthma and manifestations of allergy in general, sinusitis, herpes and viral hepatitis, Herpes Zoster and viral diseases in general, characterized in that it includes in aqueous solution :

(1) at least one organo-silicon compound represented by either of the two formulas :

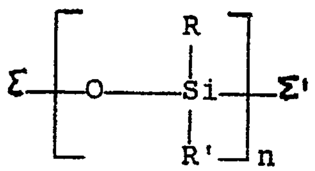

or

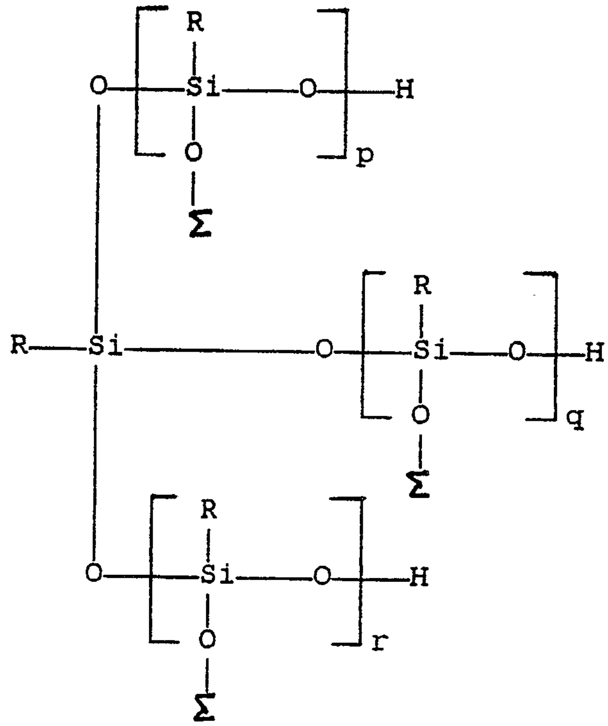

(A) (B)

where :

- n is a whole number between 1 and 1000 and, preferably, between 1 and 50;
- p, q, r are whole numbers between 0 and 1000 and, preferably, between 0 and 60 ;
- R and R' representing, independently of one another, a saturated or unsaturated linear, branched or cyclic group and possibly including heteroatoms, or an aromatic, heteroaromatic, arylaliphatic, or heteroarylaliphatic group, possibly substituted by functional groups, it being possible also for R to be OH, OR or $OSiR_3$;
- Σ being R or H or $SiR_3$ ;
- Σ' being chosen from R and more particularly $CH_3$ or OR, or OH or $OSiR_3$, (where R is such as is described above), or alkali metal or addition salts with a pharmaceutically acceptable acid, of said compound,

  the organo-silicon compound(s) being used at a concentration in water varying between $10^{-5}$ and $10^{-1}$ mol/l and more particularly between $10^{-4}$ and $10^{-2}$ mol/l, and

(2) sodium hyposulfite ($Na_2S_2O_3$, $5H_2O$) or magnesium hyposulfite, the ratio of (1) to (2) being 5 to 50 g of hyposulfite per litre of organo-silicon solution, preferably about 20 g/litre of organo-silicon solution.

2. Composition as claimed in claim 1, characterized in thant it includes in addition one or several organic acids or their salts.

3. Composition as claimed in either claims 1 or 2, characterized in that it consists of a unique product containing the organo-silicon compound and the hyposulfite in a single solution.

4. Composition as claimed in either claims 1 or 2, characterized in that it consists of a two-component product containing the organo-silicon and the hyposulfite separately which are intended to be simultaneously administered.

5. Composition as claimed in any of the claims 1 to 4, characterized in that it is in the form of unit doses, in bottles or ampoules, containing about 10 $cm^3$ of said composition.

6. Composition as claimed in any of the claims 1 to 5, characterized in that it has a pH adjusted to between 3 and 7, preferably between 3 and 5.

**Ansprüche**

1. Zusammensetzung zur therapeutischen Verwendung, insbesondere geeignet zur Behandlung von Asthma und allergischen Phenomenen allgemein, von Sinusitis, von Herpes und Virushepatitis, von Gürtelrose und Viruserkrankungen allgemein, dadurch **gekennzeichnet,** daß sie in wässriger Lösung
(1) wenigstens eine Organosilicatverbindung entsprechend einer der beiden Formeln:

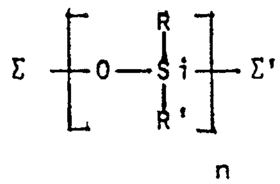

oder

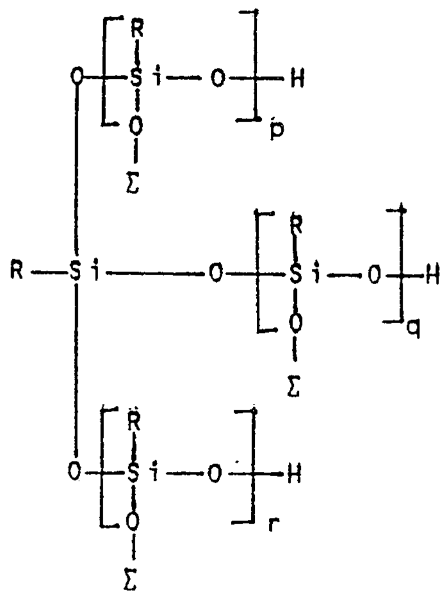

(A) (B)

in denen
- n eine ganze Zahl zwischen 1 und 1000 und vorzugsweise zwischen 1 und 50 ist,
- p, q, r ganze Zahlen zwischen 0 und 1000 und vorzugsweise zwischen 0 und 60 sind,
- R und R' unabhängig voneinander eine lineare, verzweigte oder zyklische, gesättigte oder ungesättigte Gruppe darstellen, die gegebenenfalls oder eine aromatisierte, heteroaromatische, arylaliphatische oder heteroaliHeteroatome phatische Gruppe gegebenenfalls substituiert durch funktionelle Gruppen enthalten, wobei R auch OH, OR oder $OSiR_3$ sein kann,
- Σ R oder H oder $SiR_3$ ist,
- Σ' ausgewählt aus R und insbesondere $CH_3$ oder OR oder OH oder $OSiR_3$ (wobei R wie oben definiert ist) ist, oder die Alkalimetallsalze oder einen Zusatz mit einer pharmazeutisch akzeptablen Säure der besagten Verbindung enthält,
wobei die Organosilicateverbindung(en) in einer Konzentration, die von $10^{-5}$ bis $10^{-1}$ mol/l und insbesondere von $10^{-4}$ bis $10^{-2}$ mol/l variiert, verwendet wird/werden, und

(2) Hyposulfit von Natrium ($Na_2S_2O_3.5H_2O$) oder Magnesium enthält, wobei das Verhältnis von (1) zu (2) 5 bis 50g Hyposulfit pro 1 Liter Organosilicatlösung und vorzugsweise etwa 20 g/Liter Organosilicatlösung beträgt.

2. Zusammensetzung nach Anspruch 1, dadurch gekennzeichnet, daß sie zudem eine oder mehrere organische Säuren oder deren Salze enthält.

3. Zusammensetzung nach einem der Ansprüche 1 oder 2, dadurch gekennzeichnet, daß sie aus einem einheitlichen Produkt enthaltend die Organosilicatverbindung und das Hyposulfit in einer einzigen Lösung besteht.

4. Zusammensetzung nach einem der Ansprüche 1 bis 2, dadurch gekennzeichnet, daß sie aus einem Zweikomponentenprodukt, das die Organisilicatverbindung und das Hydrosulfit, die zur gleichzeitigen Anwendung bestimmt sind, getrennt enthält.

5. Zusammensetzung nach einem der Ansprüche 1 bis 4, dadurch gekennzeichnet, daß sie in Form von Einheitsdosen in Flakons oder Ampullen enthaltend etwa 10 $cm^3$ der Zusammensetzung vorliegt.

6. Zusammensetzung nach einem der Ansprüche 1 bis 5, dadurch gekennzeichnet, daß sie einen zwischen 3 und 7, insbesondere zwischen 3 und 5 eingestellten pH-Wert aufweist.